# EUROPÄISCHE PATENTSCHRIFT

(11) **EP 1 982 696 B1**
(45) Veröffentlichungstag und Bekanntmachung des Hinweises auf die Patenterteilung: **19.05.2010**
(21) Anmeldenummer: 07007981.9
(22) Anmeldetag: 19.04.2007
(51) Int. Cl.: A61K 9/06, A61K 31/44, A61K 31/785, A61K 47/34, A61K 9/00, A61P 17/02, A61P 31/02

(54) **Gel zur Behandlung von Wunden**
Gel for treating wounds
Gel destiné au traitement de blessures

(43) Veröffentlichungstag der Anmeldung: 22.10.2008
(73) Patentinhaber: Dr. August Wolff GmbH & Co. KG Arzneimittel, 33611 Bielefeld (DE)
(72) Erfinder: Abels, Christoph, 33617, Bielefeld (DE); Knie, Ulrich, 32105 Bad Salzuflen (DE); von der Höh, Annette, 33729, Bielefeld (DE)
(74) Vertreter: Keller, Günter

(56) Entgegenhaltungen:
- EP-A1- 0 672 422
- FR-A1- 2 585 575
- GB-A- 2 362 100
- US-A- 4 849 418
- US-A1- 2006 051 385
- NALBANDIAN R M ET AL: "PLURONIC F-127 GEL PREPARATION AS AN ARTIFICIAL SKIN IN THE TREATMENT OF THIRD-DEGREE BURNS IN PIGS" JOURNAL OF BIOMEDICAL MATERIALS RESEARCH, WILEY, NEW YORK, NY, US, Bd. 21, Nr. 9, September 1987 (1987-09), Seiten 1135-1148, XP002915634 ISSN: 0021-9304
- NALBANDIAN R M ET AL: "Artificial skin. II. Pluronic F-127 Silver nitrate or silver lactate gel in the treatment of thermal burns." November 1972 (1972-11), JOURNAL OF BIOMEDICAL MATERIALS RESEARCH NOV 1972, VOL. 6, NR. 6, PAGE(S) 583 - 590 , XP002451837 ISSN: 0021-9304 * Zusammenfassung * * Seite 1137, Zeilen 32-37 * * Seite 1138, Zeilen 8-18 * * Seite 1147, Zeilen 16-24 *

## Beschreibung

Die vorliegende Erfindung betrifft ein antiseptisches Gel auf der Basis von Poloxamer 407, das Octenidin und/oder Polihexanid als Wirkstoff beinhaltet und eine sehr hohe antibakterielle Wirksamkeit aufweist. Die erfindungsgemäßen Zusammensetzungen stellen daher ein ideales Gel zur Behandlung von Wunden zur Verfügung, das zudem höchste Anforderungen bezüglich der Herstellung, Lagerung und Anwendung erfüllt. Des Weiteren sind die erfindungsgemäßen Gele zusätzlich besonders vorteilhaft, wenn sie einen sprunghaften Viskositätsanstieg in einem Temperaturbereich zwischen 18°C und 25°C aufweisen. Als "sprunghaft" im Sinne der vorliegenden Erfindung wird bevorzugt ein Viskositätsanstieg von mindestens 20 Pa s innerhalb von 2°C angesehen.

Die Bedeutung antiseptischer Lokaltherapeutika in der Wundversorgung ist unbestritten. In der Vergangenheit wurden häufig Lokalantibiotika wie Chlortetracyclin, Framycetin, Gentamycin, Neomycin / Bacitracin, Nitrofurazon und Thyrothicin eingesetzt. Auf diese Stoffgruppe wird heutzutage aufgrund von Lücken im Wirksamkeitsspektrum, Resistenzentwicklung, Wundheilungshemmung, lokalen Hautreaktionen und Kontaktallergien weitgehend verzichtet. Auch andere Wundantiseptika wie 8-Chinolinolsulfat, Chloramin-T, Chlorhexidin, Ethacridinlactat, Kaliumpermanganat und Quecksilberpräparate gelten als veraltet, da sie häufig zu Lokalreaktionen, Verfärbungen von Wunde und Wäsche sowie teilweise zu ausgeprägter Wundheilungshemmung führen. Von Bedeutung sind noch PVP (Povidon)-lod-Präparate, deren wesentliche Nachteile in den schlecht auswaschbaren Verfärbungen, dem Eiweißfehler und Kontraindikationen im Bereich Schwangerschaft, Stillzeit und Schilddrüsenfunktionsstörung liegen.

An zeitgemäße, lokalwirksame, antiseptische Therapeutika werden folgende Anforderungen gestellt: Nicht färbend, schmerzfrei applizierbar, nicht systemisch wirksam, sichere Wirkung in kurzer Zeit, allergologische Unbedenklichkeit, breites Wirkungsspektrum, keine Resistenzgefahr.

Polihexanid gehört zur Gruppe der Biguanide. Der kationische Biguanidanteil interagiert mit sauren Phospholipidpartialstrukturen der bakteriellen Zellmembran und erhöht deren Permeabilität (Eifler-Bollen et al., Hautarzt (2005) 56: 752). Unter Verlust von zellulären Zytoplasmabestandteilen, vor allem Kaliumionen, kommt es zum Zelluntergang. Polihexanid besitzt eine große therapeutische Breite, aufgrund der selektiven Wirkung gegenüber sauren Lipiden bakterieller Zellmembranen. Das breite mikrobizide Spektrum umfasst *Staph. aureus, Strept. faecalis, Strept. lactis, Bacillus subtilis, Escherichia coli, Enterobacter cloacae* und *Saccharomyces spp.* Darüber hinaus ist die Wirksamkeit gegenüber Chlamydia trachomatis beschrieben. Die mikrobizide Wirkung tritt erreger- und konzentrationsabhängig innerhalb von 5-20 Minuten ein. Im Vergleich zu anderen Lokalantiseptika besitzt Polihexanid keinen Eiweißfehler und wird hinsichtlich der therapeutischen Breite und Gewebeverträglichkeit als sehr günstig eingeschätzt. Polihexanid wird sowohl zur Prophylaxe von infektionsgefährdeten Wunden als auch zur Behandlung infizierter, akuter und chronischer Wunden angewendet.

Octenidindihydrochlorid (hier als Octenidin bezeichnet) ist ein oberflächenaktiver Wirkstoff und gehört zur Stoffklasse der kationischen Bispyridiniumalkane mit Wirkung auf die Zellmembran. Er wird für die Antiseptik 0,1%-ig in Kombination mit 2% Phenoxyethanol in Form von Lösungen eingesetzt. Das Wirkungsspektrum umfasst grampositive und gramnegative Bakterien, Pilze sowie eine Reihe von Virusarten. Bei der Anwendung auf Wunden war bisher keine Resorption nachweisbar, so dass nach derzeitigem Stand des Wissens resorptiv-toxische Risiken auszuschließen sind.

Nicht nur die antiseptische Wirkung und die lokale Verträglichkeit allein sind entscheidend für die Akzeptanz eines Produktes beim Arzt, Pflegepersonal und Patienten. Idealerweise soll ein Wundantiseptikum auf der Wunde gut haften, um ein Herausfließen bei Lageveränderung des Patienten zu vermeiden. Es soll transparent sein, um eine Wundinspektion zu erlauben und die Wunde feucht zu halten. Des Weiteren soll es leicht abwaschbar sein, um die Wunde bei einem Verbandwechsel schmerzfrei reinigen zu können.

Eine besonders wichtige Eigenschaft eines idealen Gels zur Behandlung von Wunden ist, dass es bei Temperaturen unterhalb der Körpertemperatur ausreichende Fließeigenschaften aufweist, damit es gut auf Wunden aufgetragen werden kann. Aufgrund der geringen Viskosität bei tiefen Temperaturen kann das Gel dann auch in Vertiefungen eindringen und diese ausfüllen. Während des Aufwärmens auf Körpertemperatur erhöht sich dann die Viskosität des Gels um die Wunde beständig zu verschließen.

Weitere Probleme, die durch die Änderung der Viskosität in Abhängigkeit der Temperatur entstehen können, sind beispielsweise eine Phasentrennung der einzelnen Bestandteile des Gels bzw. ein unerwünschtes Abscheiden des Wirkstoffes. Insbesondere weisen die bisher bekannten Gele einen geringeren Wassergehalt als die erfindungsgemäßen Gele auf und sind daher unterhalb der Raumtemperatur weniger flüssig.

Von Polihexanid und Octenidin sind Lösungen und Gele im Handel erhältlich. Beispielsweise vertreibt die Firma PRONTOMED GmbH ein Gel auf Basis von Hydroxyethylcellulose mit Polyhexanid als Konservierungsmittel (Prontosan^{®}D). Diese Gele erfüllen jedoch die Anforderungen an ein ideales Gel zur Behandlung von Wunden nicht. Insbesondere haben diese Gele keine Fließgrenze und fließen deshalb wieder aus der Wunde heraus.

Gele auf Basis von Poloxamer, die den Wirkstoff Octenidin oder Polihexanid enthalten, sind bislang nicht erhältlich. Poloxamere sind Blockpolymerisate aus einem lipophilen und zwei hydrophilen Molekülteilen mit oberflächenaktiven Eigenschaften, die in der Lage sind, transparente Tensidgele zu bilden (Nürnberg et al., (1989) 129: 2183). Poloxamere werden durch Copolymerisation von Propylenoxid und Ethylenoxid gewonnen. Das besondere an diesen Tensidgelen ist ihre thermotrope Sol-Gel-Umwandlung. Sie verflüssigen sich im Kalten und bilden bei höheren Temperaturen streichfähige Gele. Die Temperatur, bei der diese Sol-Gel-Umwandlung stattfindet, ist abhängig vom Poloxamertyp und den anderen Bestandteilen wie beispielsweise Wasser, Propylenglykol und Glycerol sowie deren Gewichtsanteilen ist die Sol-Gel-Umwandlungstemperatur im Bereich von 20 bis 30 °C.

Insbesondere ist die Verwendung von Glycerol anstelle von Propylenglykol vorteilhaft, da Glycerol einen größeren Viskositätsanstieg bewirkt. Unterhalb dieser Temperatur kann die flüssige Lösung auf die Wunde aufgegossen werden, durch die Sol-Gel-Umwandlung geliert sie dann bei der Erwärmung durch die Körpertemperatur zu einem transparenten Gel. Tiefe Wundhöhlen werden so komplett gefüllt. Diese Gele sind pH-neutral und sehr gut haut- und schleimhautverträglich.

Antibiotika- und Silbernitrathaltige Poloxamergele sind aus der Literatur bekannt (Faulkner et al., Amer. J. Emergency Med. (1997) 15: 20-24; Nalbandian et al., J. Biomed. Mater. Res. (1972) 6: 583; Nalbandian et al., J. Biomed. Mater. Res. (1987) 21: 1135).

Kommerzielle Anwendung haben diese antiseptischen Poloxamergele in industriell hergestellten Fertigarzneimitteln bisher nicht gefunden. Teilweise, z. B. bei Silbersulfadiazin-haltigen Gelen, ist dies auf die Instabilität durch suspendierte Wirkstoffe zurückzuführen, die bei niedrigen Temperaturen, wenn die Zubereitung flüssig ist, sedimentieren und es dabei zu einer Entmischung des Präparates kommt. Erst die erfindungsgemäße Verwendung von Octenidin bzw. Polihexanid in Kombination mit der erfindungsgemäßen Menge an Poloxamer ermöglichte die Herstellung von geeigneten antiseptischen Gelen.

US 2006/0051385 offenbart antimikrobielle Zusammensetzungen, die eine Vielzahl an Tensiden enthalten können, wobei in dieser Liste auch Poloxamer erwähnt ist (US 2006/0051385, Seite 2, [0013] und Seite 3 [0023]). Für diese Zusammensetzungen wird eine Vielzahl an kationischen, antiseptischen Wirkstoffen als geeignet beschrieben (US 2006/0051385, Seite 28, [0017]. Jedoch ist beispielsweise die vorteilhafte Verwendung eines Gels auf Poloxamerbasis, das einen sprunghaften Viskositätsanstieg aufweist, nicht erwähnt.

Weitere Gele zur Behandlung von Wunden werden in WO 01/85845 offenbart. Die in diesem Dokument verwendeten Gele zur Behandlung von Wunden sind auf der Basis von Poloxamer. Gemäß der Lehre der WO 01/85845 ist die Anwesenheit eines Poloxamers vorteilhaft, um die Viskosität der Gele bei Kontakt mit Wundsekreten aufrecht zu erhalten (WO 01/85845, Seite 2, 3. Absatz). Als geeignete Wirkstoffe für diese Gele offenbart die WO 01/85845 die Verwendung von Metronidazol, Lidocain und Teebaumöl in den Beispielen. Die Verwendung von Octenidin und Polihexanid und die spezifischen Probleme dieser Wirkstoffe werden in diesem Dokument nicht behandelt. Des Weiteren ist in Figur 1 von WO 01/85845 die Viskosität des Gels aus Beispiel 1 (ohne aktiven Wirkstoff) in Abhängigkeit der Temperatur dargestellt. In Figur 1 von WO 01/85845 ist ein Viskositätsanstieg des Gels zwischen 26°C und 31 °C zu erkennen, wobei die Zusammensetzung unterhalb von 25°C nicht flüssig ist.

EP 0 672 422 berichtet von antiinflammatorischen und analgetischen Hautgelen, die nicht steroide Propionsäurederivate als aktive Inhaltsstoffe aufweisen (EP 0 672 422, Seite 2, Zeilen 5/6). Hierbei werden Gelzusammensetzungen auf der Basis von Poloxamer eingesetzt. Diese Gele weisen jedoch keinen sprunghaften Viskositätsanstieg auf.

Eine Aufgabe der Erfindung besteht darin, ein Wundantiseptikum auf der Basis eines Gels zu entwickeln, das die oben aufgeführten Anforderungen an ein ideales Gel zur Behandlung von Wunden erfüllt und eine sehr hohe antiseptische Wirkung aufweist.

Diese Aufgabe wurde dadurch gelöst, dass die antiseptischen Wirkstoffe Octenidin und/ oder Polihexanid in einem Poloxamergel mit den erfindungsgemäßen Mengen an Poloxamer 407 eingesetzt werden.

Die vorliegende Erfindung betrifft ein antiseptisches Gel auf der Basis von Poloxamer 407, umfassend Octenidin und/ oder Polihexanid, weiter bevorzugt Octenidin als Wirkstoff, dadurch gekennzeichnet, dass das Gel zwischen 16 Gew.-% und weniger als 20 Gew.-%, bevorzugt zwischen 16 Gew.-% und 19 Gew.-%, weiter bevorzugt zwischen 17 Gew.-% und 19 Gew.-%, noch weiter bevorzugt zwischen größer als 17,5 Gew.-% und 19 Gew.-%, weiter bevorzugt zwischen 18 Gew.-% und 19 Gew.-% Poloxamer 407, jedoch kein weiteres Poloxamer aus der Gruppe bestehend aus Poloxamer 108, Poloxamer 182, Poloxamer 188, Poloxamer 237, Poloxamer 331 und Poloxamer 338, enthält. Gewichtsprozentangaben beziehen sich auf das fertige Produkt. Weiter bevorzugt enthält das antiseptische Gel außer Poloxamer 407 kein weiteres Poloxamer.

In einer weiter bevorzugten Ausführungsform der Erfindung weist das Gel zusätzlich in dem Temperaturbereich zwischen 18°C und 25°C, weiter bevorzugt zwischen 18°C und 24°C, weiter bevorzugt zwischen 19°C und 21°C, innerhalb von 2°C, noch weiter bevorzugt innerhalb von 1,5°C und am meisten bevorzugt innerhalb von 1°C einen sprunghaften Viskositätsanstieg auf. Bevorzugt wird die Messung des Viskositätsanstiegs mit einem Platte-Kegel Rheometer (Kegel: 50 mm Durchmesser, Winkel 1°) und einer Scherrate von 5 1/s bei einer Heizrate von 3 °C pro Minute über den Bereich von 15 °C bis 30 °C durchgeführt.

Eine besonders bevorzugte Ausführungsform der Erfindung betrifft ein antiseptisches Gel, worin zusätzlich Wasser und Glycerin als Bestandteile enthalten sind. In einer weiter bevorzugten Ausführungsform des Gels ist nur Octenidin und/ oder Polihexanid, Poloxamer 407, Glycerin und Wasser enthalten. Weiter bevorzugt wird nur Octenidin verwendet.

Eine weiter bevorzugte Ausführungsform betrifft ein antiseptisches Gel, worin der Gehalt an Octenidin und/ oder Polihexanid zwischen 0,01 Gew.-% und 2 Gew.-% liegt. Bei Verwendung beider Wirkstoffe bezieht sich die Mengenangabe in Gew.-% auf die Gesamtmenge an Wirkstoffen. Bevorzugt wird nur Octenidin als Wirkstoff verwendet.

In einer anderen Ausführungsform des antiseptischen Gels beträgt der Gehalt an Octenidin und/ oder Polihexanid zwischen 0,05 Gew.-% und weniger als 0,1 Gew.-%. Weiter bevorzugt wird nur Octenidin verwendet.

Eine weiter bevorzugte Ausführungsform der Erfindung betrifft ein antiseptisches Gel, worin zwischen 7 Gew.-% und 15 Gew.-%, weiter bevorzugt zwischen 9 Gew.-% und 12 Gew.-% und noch weiter bevorzugt zwischen 10 Gew.-% und 12 Gew.- % Glycerin enthalten sind.

Eine noch weiter bevorzugte Ausführungsform der Erfindung betrifft ein antiseptisches Gel, worin mindestens 68 Gew.-% Wasser, 17 Gew.-% bis 20 Gew.-% Poloxamer, 7-10 Gew.-% Glycerin und 0,01 Gew.-% bis 2 Gew.-% Octenidin oder Polihexanid enthalten sind. Weiter bevorzugt sind zwischen 0,05 Gew.-% und weniger als 0,01 Gew.-% Polihexanid oder Octenidin enthalten. Bevorzugt ist Octenidin in den angegebenen Mengen enthalten.

Des Weiteren betrifft die vorliegende Erfindung die Herstellung der oben beschriebenen antiseptischen Gele.

Bevorzugterweise erfolgt das Verfahren zur Herstellung eines antiseptischen Gels unter aseptischen Bedingungen. Besonders bevorzugt wird keine Autoklavierung oder Bestrahlung durchgeführt.

Die vorliegende Erfindung betrifft zudem die Verwendung von Octenidin oder Polihexanid zur Herstellung eines antiseptischen Gels zur Behandlung von Wunden. Die Behandlung von Wunden umfasst hierbei bevorzugt das topische Auftragen des antiseptischen Gels auf Wunden von Patienten.

Die vorliegende Erfindung betrifft auch die Verwendung eines Gemisches aus 17 Gew.-% bis 25 Gew.-% Poloxamer 407, 15 - 17 Gew.-% Glycerin und mehr als 58 Gew.-% Wasser zur Erzeugung eines sprunghaften Viskositätsanstiegs eines Gels in einem Temperaturbereich zwischen 18°C und 25°C, weiter bevorzugt zwischen 19°C und 21 °C, wobei der sprunghafte Viskositätsanstieg innerhalb von 2°C erfolgt und wobei außer Poloxamer 407 kein weiteres Poloxamer enthalten ist.

Die erfindungsgemäße Gelzusammensetzung mit einem sprunghaften Viskositätsanstieg in dem Bereich zwischen 18°C und 25°C hat sich als besonders geeignet für ein ideales Gel zur Behandlung von Wunden erwiesen, obwohl dieser Temperaturbereich erheblich unterhalb der Körpertemperatur von 37°C liegt. Insbesondere würde ein Fachmann erwarten, dass das Auftragen des Gels auf die Haut durch den Viskositätsanstieg bei relativ geringen Temperaturen durch ein zu frühes "Verfestigen" negativ beeinflusst wird. Jedoch zeigt es sich, dass gerade der erfindungsgemäße Bereich des sprunghaften Viskositätsanstiegs vorteilhaft gegenüber beispielsweise einem höheren Bereich von 26°C bis 31°C, wie in WO 01/785845 offenbart, ist.

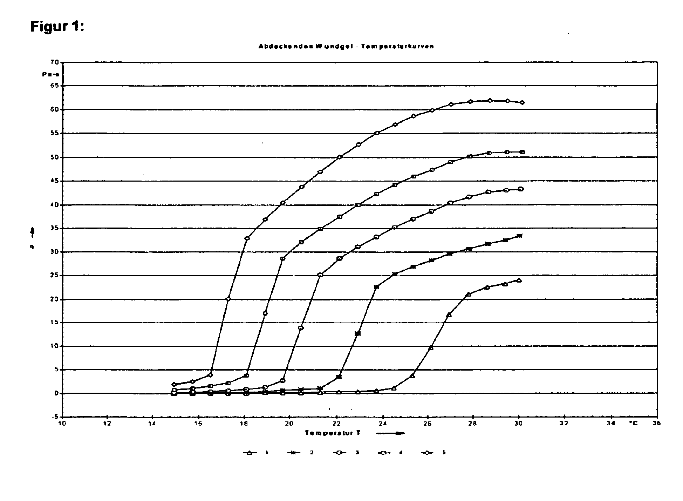

Diesbezüglich wurde überraschenderweise festgestellt, dass der Viskositätsanstieg der erfindungsgemäßen Zusammensetzungen mit dem erfindungsgemäßen Poloxameranteil in dem Bereich zwischen 18°C und 25°C liegt und größer ist, als der Viskositätsanstieg von bekannten Zusammensetzungen. Figur 1 zeigt, dass der Viskositätsanstieg mit abnehmendem Gehalt an Poloxamer geringer ausfällt und daher ist ein zu geringer Gehalt an Poloxamer nicht vorteilhaft. Des Weiteren fällt mit abnehmendem Poloxamergehalt nicht nur der Viskositätsanstieg geringer aus, sondern der Viskositätsanstieg erfolgt auch unerwünschterweise bei höheren Temperaturen.

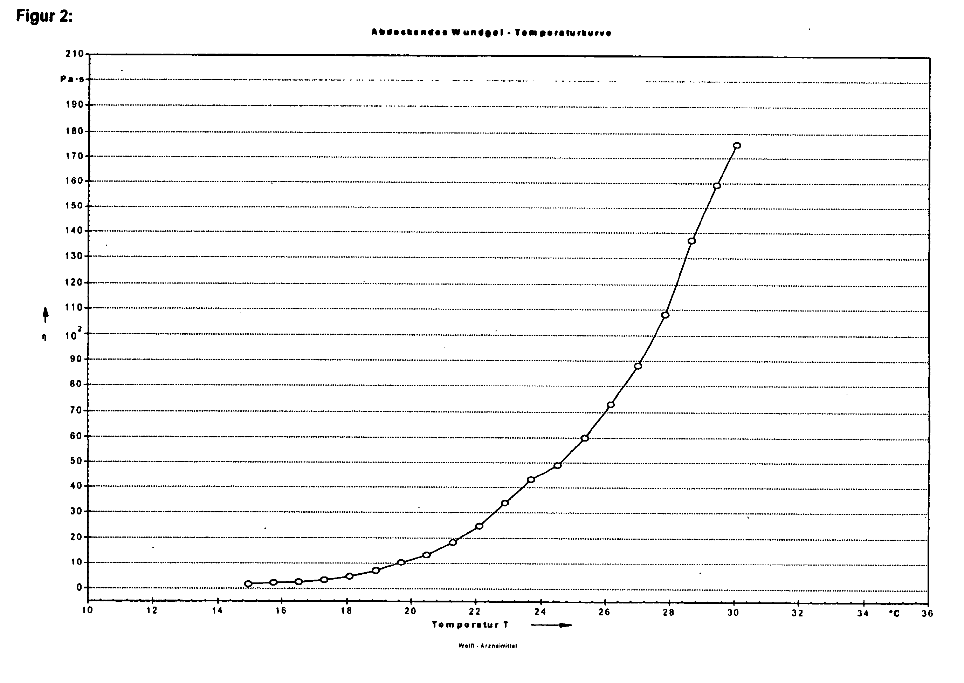

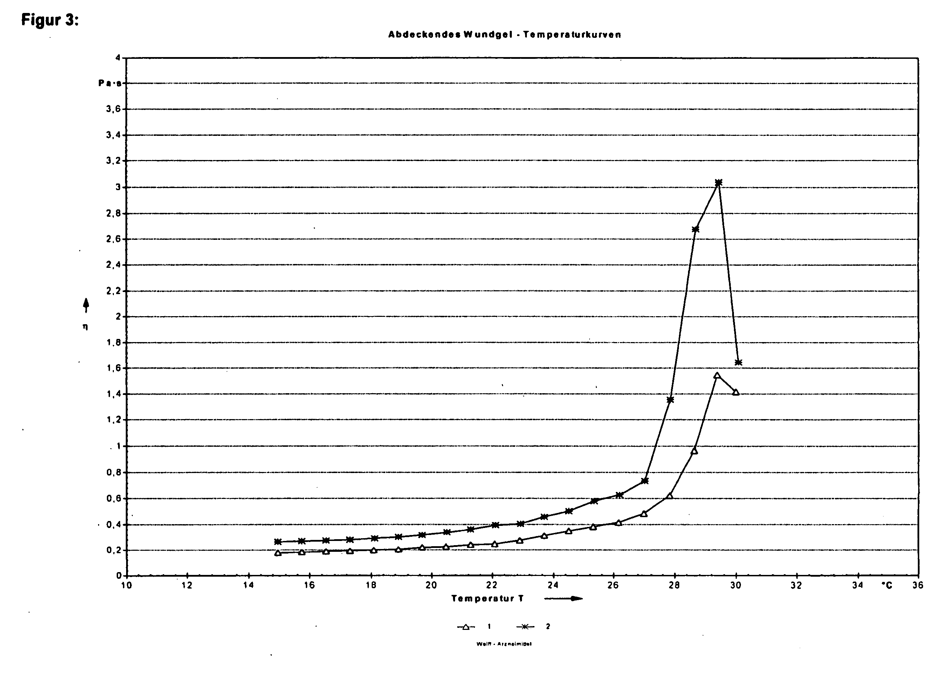

Des Weiteren wurde auch überraschenderweise festgestellt, dass die Verwendung von 18 Gew.-% Poloxamer 407 (Handelsname Pluronic F127^{®}) zusammen mit 20 Gew.-% Poloxamer 188 (Handelsname Pluronic F68^{®}) nicht geeignet ist, den gewünschten sprunghaften Viskositätsanstieg zu erzeugen (siehe Figur 2). Auch eine Veränderung der Mengenverhältnisse führt nicht zu den gewünschten Eigenschaften. Figur 3 zeigt, dass die Verringerung der Menge an Poloxamer 188 zu noch ungeeigneteren Gelen führt.

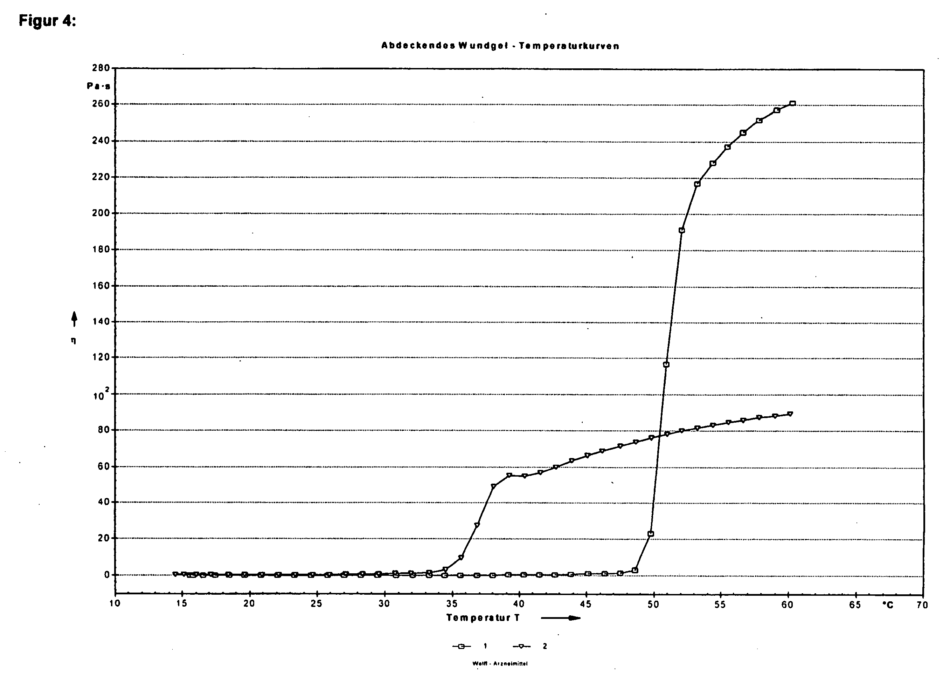

Des Weiteren zeigt sich überraschenderweise auch, dass die Verwendung von Poloxamer 188 oder Poloxamer 388 alleine ebenfalls nicht geeignet ist, die erfindungsgemäße Aufgabe zu lösen. Figur 4 zeigt diesbezüglich die Verwendung von 30 Gew.-% Poloxamer 188 bzw. von 30 Gew.-% Poloxamer 388 (Handelsname Pluronic F108^{®}). Poloxamer 188 alleine bildet bei 17 Gew.-%, 18 Gew.-% und 19 Gew.-% keine Gele und ergibt bei 50 Gew.-% eine pastöse Masse. Des Weiteren ist Poloxamer 388 alleine bei 18 Gew.-% und 20 Gew.-% flüssig und bildet keine Gele. Die erfindungsgemäße Aufgabe wird daher nur durch die Verwendung von Poloxamer 407 gelöst.

Wie oben erwähnt, kommt der Viskosität im Bereich der Körpertemperatur eine besondere Bedeutung zu, da die Eigenschaften nach Auftragen auf die Wunde bzw. die Haltbarkeit des Gels von großem Interesse sind. Diesbezüglich haben sich die erfindungsgemäßen Gele ebenfalls als hervorragend geeignet erwiesen. Insbesondere weisen die erfindungsgemäßen Gele eine hohe Viskosität und Festigkeit auf und ermöglichen damit einen sehr guten mechanischen Schutz der Wunde.

Unerwarteterweise zeigen die erfindungsgemäßen Gele zudem eine sehr hohe antibakterielle Wirksamkeit bei geringer Wirkstoffkonzentration. So erfüllt das erfindungsgemäße Gel schon bei einem Wirkstoffgehalt von 0,05 Gew.-% Octenidin die Anforderungen der DIN EN 1276 (vergleiche Beispiel 3), ohne dass zusätzlich Phenoxyethanol verwendet wird. Überraschenderweise zeigte Octenidin eine noch höhere Wirksamkeit als Polihexanid.

Zudem führt die erfindungsgemäße Verwendung von Octenidin und Polihexanid in einem Poloxamergel zu sedimentationsstabilen Gelen und ermöglicht erstmals die Herstellung von antiseptischen Gelen auf der Basis von Poloxamer. Ein Vorteil des sprunghaften Viskositätsanstiegs besteht darin, dass das Gel leicht und bequem aufgetragen werden kann, sich dann aber schnell verfestigt.

Die erfindungsgemäße Aufgabe wird durch die nachfolgend beschriebenen antiseptischen Gele gelöst.

Die erfindungsgemäßen Gele sind auf der Basis von Poloxamer 407, wobei kein weiteres Poloxamer aus der Gruppe bestehend aus: Poloxamer 108, Poloxamer 182, Poloxamer 188, Poloxamer 237, Poloxamer 331, Poloxamer 338, enthalten ist. Noch weiter bevorzugt ist, dass außer Poloxamer 407 kein weiteres Poloxamer enthalten ist. Noch weiter bevorzugt ist, dass außer Poloxamer 407 kein zusätzliches Tensid enthalten ist. Am meisten bevorzugt ist, dass außer Poloxamer 407 kein weiteres Poloxamer und kein weiteres Tensid enthalten ist.

Der Ausdruck "Poloxamer", so wie hier verwendet umfasst Blockpolymerisate aus einem lipophilen (z.B. Polypropylenglykol) und zwei hydrophilen Molekülteilen (z.B. Polyethylenglykol), die durch Copolymerisation von Propylenoxid und Ethylenoxid gewonnen werden. Die bekannten Poloxamere werden meist als "Poloxamer" in Verbindung mit einer Nummer bezeichnet.

Der Ausdruck "Tensid", so wie hier verwendet umfasst oberflächenaktive Verbindung aus der Gruppe bestehend aus Sulfonaten, Sulfaten, Phosphonaten, Phosphaten, kationischen Tensiden oder Gemischen davon.

Bevorzugt weist das Gel einen Gehalt an Poloxamer 407 zwischen 16 Gew.-% und weniger als 20 Gew.-%, bevorzugt zwischen 16 Gew.-% und 19 Gew.-%, weiter bevorzugt zwischen 17 Gew.-% und 19 Gew.-%, noch weiter bevorzugt zwischen größer als 17 Gew.-% und 19 Gew.-%, weiter bevorzugt zwischen 18 Gew.-% und 19 Gew.-% auf.

Zusätzlich ist es bevorzugt, dass die erfindungsgemäße Gelzusammensetzung Wasser enthält. Bevorzugterweise wird ein Wassergehalt von mindestens 58%, weiter bevorzugt mindestens 68% verwendet.

Zusätzlich ist es bevorzugt, dass das erfindungsgemäße Gel Glycerin enthält. Besonders bevorzugt werden 7-15 Gew.-%, weiter bevorzugt 9-12 Gew.-%, noch weiter bevorzugt 10-12 Gew.-% Glycerin, eingesetzt.

Das erfindungsgemäße Gel enthält die aktiven Wirkstoffe Polihexanid und/oder Octenidin, bevorzugterweise Octenidin und besonders bevorzugt wird ein Gemisch der beiden Wirkstoffe eingesetzt. Bevorzugterweise beträgt der Gehalt der einzelnen aktiven Wirkstoffe zwischen 0,01 Gew.-% und 2 Gew.-%, weiter bevorzugt zwischen 0,01 Gew.-% und 0,1 Gew.-% und am meisten bevorzugt zwischen 0,05 Gew.-% und weniger als 0,1 Gew.-%. Bevorzugt ist ein Gemisch der beiden Wirkstoffe, wobei die Wirkstoffe jeweils in den vorstehend genannten Mengen verwendet werden.

Zusätzlich ist es bevorzugt, dass die erfindungsgemäßen Gele einen sprunghaften Viskositätsanstieg in dem Temperaturbereich zwischen 18°C und 25°C aufweisen, wobei der sprunghafte Viskositätsanstieg innerhalb von 2°C erfolgt. Der Ausdruck "innerhalb von 2°C" bedeutet hierbei, dass der sprunghafte Viskositätsanstieg innerhalb eines Bereichs von 2°C erfolgt. Besonders bevorzugt sind antiseptische Gele, die einen sprunghaften Viskositätsanstieg zwischen 19°C und 21 °C aufweisen.

Des Weiteren ist es zusätzlich bevorzugt, dass das Gel Verbindungen enthält, die die antibakterielle Wirkung von Octenidin und/ oder Polihexanid verstärken bzw. beschleunigen. Geeignete Verbindungen hierfür können beispielsweise aus der Gruppe ausgewählt werden, bestehend aus: alpha-Hydroxysäuren, beta-Hydroxysäuren oder andere Carbonsäuren, (C₁-C₄)alkyl-Carbonsäuren, (C₆-C₁₂)aryl-Carbonsäuren, (C₆-C₁₂)aralkyl-Carbonsäuren, (C₆-C₁₂)alkaryl-Carbonsäuren, Gelatorverbindungen, phenolischen Verbindungen (wie bestimmte Antioxidanzien und Parabene), (C₁-C₁₀)-Monohydroxyalkohol, oder Glykolether bzw. Gemische davon. Die alpha-Hydroxysäuren bzw. beta-Hydroxysäuren und die anderen Carbonsäuren liegen hierbei bevorzugterweise in ihrer protonierten Form in dem Gel vor. Derartige Verbindungen können beispielsweise in einer Menge von 0,01 Gew.-% bis 2 Gew.-% verwendet werden.

Damit sich eine Zusammensetzung mit insgesamt 100 Gew.-% ergibt, kann der Wassergehalt bei Verwendung zusätzlicher Bestandteile (außer Glycerin, Poloxamer 407 und Wirkstoffen) entsprechend verringert werden.

Die Herstellung der erfindungsgemäßen Gele kann mit Methoden erfolgen, die dem Fachmann bekannt sind. Beispielsweise kann die Herstellung des Gels durch Auflösen des Poloxamers 407 bei Temperaturen oberhalb von 70°C oder aber bei ca. 5 - 10 C° erfolgen.

Bevorzugterweise erfolgt die Herstellung des Gels bei tiefen Temperaturen, da dadurch keine Zersetzung des Wirkstoffs eintreten kann. Die Herstellung eines erfindungsgemäßen antiseptischen Gels ist auch in Beispiel 1 beschrieben.

Die Bestimmung der Viskosität in Abhängigkeit der Temperatur kann ebenfalls mit Messmethoden durchgeführt werden, die dem Fachmann bekannt sind. Der Begriff "sprunghafter Viskositätsanstieg" bezieht sich bevorzugterweise auf einen Viskositätsanstieg innerhalb von 2°C, weiter bevorzugt innerhalb von 1,5°C und weiter bevorzugt innerhalb von 1°C von mindestens 20 Pa s, weiter bevorzugt von mindestens 25 Pa s, noch weiter bevorzugt 27 Pa s, weiter bevorzugt von mindestens 30 Pa s, weiter bevorzugt von mindestens 35 Pa s, weiter bevorzugt von mindestens 40 Pa s, noch weiter bevorzugt von mindestens 45 Pa s und am meisten bevorzugt von mindestens 50 Pa s. Der Viskositätsanstieg bezieht sich hierbei auf die größte Differenz der Werte für die Viskosität in dem jeweiligen Bereich, wobei der größte Wert für die Viskosität bei einer höheren Temperatur als der niedrigste Wert für die Viskosität auftreten muss. Da Differenzwerte gemessen werden, ist jedes bekannte Absolut-Messverfahren für die Messung der Viskosität geeignet. Relativ-Messsysteme wie beispielsweise ein Brookfield-Viskosimeter sind nicht geeignet. Besonders bevorzugt ist die Verwendung eines Rheometers in Form einer Platte-Kegelapparatur (50 mm Durchmesser, Winkel 1°) und einer Scherrate von 5 1/s bei einer Heizrate von 3 °C pro Minute über den Bereich von 15 °C bis 30 °C.

### Beschreibung der Figuren

**Figur 1** zeigt den Viskositätsanstieg von verschiedenen Gelen mit unterschiedlichen Gehalten an Poloxamer (16 Gew.-% bis 20 Gew.-%). Die in der nachfolgenden Tabelle 1 angegebenen Gele werden in Figur 1 durch unterschiedliche Symbole dargestellt.

**Figur 2** zeigt den Viskositätsanstieg eines Gels mit 18 Gew.-% Poloxamer 407 und 20 Gew.-% Poloxamer 188. Die nachfolgende Tabelle 2 gibt die Zusammensetzung dieses Gels an.

**Figur 3** zeigt den Viskositätsanstieg von verschiedenen Gelen mit Poloxamer 407 und unterschiedlichen Gehalten an Poloxamer 188. Die in der nachfolgenden Tabelle 3 angegebenen Gele werden in Figur 3 durch unterschiedliche Symbole dargestellt.

**Figur 4** zeigt den Viskositätsanstieg von zwei verschiedenen Gelen mit Poloxamer 188 oder Poloxamer 388. Die in der nachfolgenden Tabelle 4 angegebenen Gele werden in Figur 4 durch unterschiedliche Symbole dargestellt.

**Tabelle 1:**

| **Inhaltsstoffe** | **Gel-Nr. 1** | **Gel-Nr. 2** | **Gel-Nr. 3** | **Gel-Nr. 4** | **Gel-Nr. 5** |
|---|---|---|---|---|---|
| Polihexanid 20%-ig | 1,50 g | 1,50 g | 1,50 g | 1,50 g | 1,50 g |
| Glycerol 85% | 10,0 g | 10,0 g | 10,00 g | 10,0 g | 10,0 g |
| Wasser, gereinigt | 72,5 g | 71,5 g | 70,50 g | 69,5 g | 68,5 g |
| Poloxamer 407 | 16,0 g | 17,0 g | 18,00 g | 19,0 g | 20,0 g |
| **Gesamtmenge** | 100,0 g | 100,0 g | 100,0 g | 100,0 g | 100,0 g |
| **Symbol in** **Fig. 1** | Δ | * | ○ | □ | ◇ |

Nur die Gele (Nummern 2, 3, 4 und 5) mit einem Poloxamergehalt von mindestens 17% weisen einen sprunghaften Anstieg der Viskosität in einem Temperaturbereich zwischen 18°C und 25°C auf.

**Tabelle 2:**

| **Inhaltsstoffe** | **Gel** |
|---|---|
| Polihexanid 20%-ig | 1,50 g |
| Glycerol 85% | 10,0 g |
| Wasser, gereinigt | 50,5 g |
| Poloxamer 407 | 18,0 g |
| Poloxamer 188 | 20,0 g |
| **Gesamtmenge** | 100,0 g |
| **Symbol in** **Fig. 2** | ○ |

**Tabelle 3:**

| **Inhaltsstoffe** | **Gel-Nr.1** | **Gel-Nr.2** |
|---|---|---|
| Polihexanid 20%-ig | 1,50 g | 1,50 g |
| Glycerol 85% | 10,0 g | 10,0 g |
| Wasser, gereinigt | 65,5 g | 50,5 g |
| Poloxamer 407 | 18,0 g | 18,0 g |
| Poloxamer 188 | 5,0 g | 10,0 g |
| **Gesamtmenge** | 100,0 g | 100,0 g |
| **Symbol in** **Fig. 3** | Δ | * |

**Tabelle 4:**

| **Inhaltsstoffe** | **Gel-Nr.1** | **Gel-Nr.2** |
|---|---|---|
| Polihexanid 20%-ig | 1,50 g | 1,50 g |
| Glycerol 85% | 10,0 g | 10,0 g |
| Wasser, gereinigt | 58,5 g | 50,5 g |
| Poloxamer 188 | 30,0 g | |
| Poloxamer 388 | | 30,0 g |
| **Gesamtmenge** | 100,0 g | 100,0 g |
| **Symbol in** **Fig. 4** | □ | ∇ |

### Beispiel 1:

Die Gele aus Figur 1 wurden wie folgt hergestellt: Polihexanid-Lösung (30%-ig), Glycerin, Wasser und Poloxamer werden zusammen in einen verschließbaren Behälter verwogen und über Nacht bei 4 - 8°C aufbewahrt. Nach vollständiger Lösung wird der Ansatz durch Rühren homogen gemischt und bei Raumtemperatur gelagert. Die Gele könne aber auch, auf andere Weise hergestellt werden, beispielsweise bei erhöhter Temperatur.

### Beispiel 2:

Ein Poloxamergel mit einem Gehalt von 0,3 Gew.-% Polihexanid, 10 Gew.-% Glycerol, 18 Gew.-% Poloxamer 407 und 71,8 Gew.-% Wasser weist einen sprunghaften Viskositätsanstieg im Temperaturbereich von 18°C bis 19°C auf.

### Beispiel 3:

Ein Poloxamergel mit einem Gehalt von 0,05 Gew.-% Octenidin Dihydrochlorid, 10 Gew.-% Glycerol, 18 Gew.-% Poloxamer 407 und 71,95 Gew.-% Wasser weist einen sprunghaften Viskositätsanstieg im Temperaturbereich von 19°C bis 21°C auf.

Dieses Poloxamergel mit 0,05 Gew.-% Octenidin Dihydrochlorid erfüllte die Anforderungen der DIN EN 1276 nach 1 Stunde und 24 Stunden Einwirkzeit. Bei einer Prüfkonzentration von 80% und einer Belastung mit 0,3% Rinderalbumin lagen die Reduktionen bei allen Testkeimen über den geforderten 5 log-Stufen.

**Tabelle 5:**

| | *S. aureus* | *E. coli* | *Ec. hirae* | *P. aeruginosa* |
|---|---|---|---|---|
| 1 h | >5,27 log | >5,29 log | >5,31 log | >5,27 log |
| 24 h | >5,27 log | >5,29 log | >5,31 log | >5,27 log |

## Patentansprüche

1. Antiseptisches Gel auf der Basis von Poloxamer 407, umfassend Octenidin und/ oder Polihexanid als Wirkstoffe, **dadurch gekennzeichnet, dass** das Gel zwischen 16 Gew.-% und weniger als 20 Gew.-% Poloxamer 407, jedoch kein weiteres Poloxamer aus der Gruppe bestehend aus, Poloxamer 108, Poloxamer 182, Poloxamer 188, Poloxamer 237, Poloxamer 331 und Poloxamer 338, enthält, wobei das Gel in dem Temperaturbereich zwischen 18°C und 25°C innerhalb eines Bereichs von 2°C einen Viskositätsanstieg von mindestens 20 Pa s aufweist.

2. Antiseptisches Gel nach einem der vorangegangenen Ansprüche, **dadurch gekennzeichnet, dass** zusätzlich Wasser und Glycerin als Bestandteile enthalten sind.

3. Antiseptisches Gel nach einem der vorangegangenen Ansprüche, **dadurch gekennzeichnet, dass** der Gehalt an Octenidin und/ oder Polihexanid zwischen 0,01 Gew.-% und 2 Gew.-% liegt,

4. Antiseptisches Gel nach einem der vorangegangenen Ansprüche, **dadurch gekennzeichnet, dass** der Gehalt an Octenidin und/ oder Polihexanid zwischen 0,05 Gew.-% und weniger als 0,1 Gew.-% liegt.

5. Antiseptisches Gel nach einem der vorangegangenen Ansprüche, **dadurch gekennzeichnet, dass** zwischen 7 Gew.-% und 15 Gew.-% Glycerin enthalten ist.

6. Antiseptisches Gel nach einem der vorangegangenen Ansprüche, **dadurch gekennzeichnet, dass** mindestens 58 Gew.-% Wasser enthalten sind.

7. Antiseptisches Gel nach einem der vorangegangenen Ansprüche, **dadurch gekennzeichnet, dass** es außer Poloxamer 407 kein weiteres Poloxamer enthält.

8. Antiseptisches Gel nach einem der vorangegangenen Ansprüche, **dadurch gekennzeichnet, dass** es aus Poloxamer 407, Octenidin und/ oder Polihexanid, Glycerin und Wasser besteht.

9. Verfahren zur Herstellung eines antiseptischen Gels nach einem der vorangegangenen Ansprüche, **dadurch gekennzeichnet, dass** man die Herstellung unter aseptischen Bedingungen durchführt.

10. Verwendung von Octenidin oder Polihexanid zur Herstellung eines antiseptischen Gels auf Poloxamerbasis zur Behandlung von Wunden, wobei das Gel zwischen 16 Gew.-% und weniger als 20 Gew.-% Poloxamer 407, jedoch kein weiteres Poloxamer aus der Gruppe bestehend aus Poloxamer 108, Poloxamer 182, Poloxamer 188, Poloxamer 237, Poloxamer 331 und Poloxamer 338 enthält, und das Gel in dem Temperaturbereich zwischen 18°C und 25°C innerhalb eines Bereichs von 2°C einen Viskositätsanstieg von mindestens 20 Pa s aufweist.

11. Verwendung eines Gemisches aus 16 Gew.-% bis 20 Gew.-% Poloxamer 407, 7 - 15 Gew.-% Glycerin und mehr als 58 Gew.-% Wasser zur Erzeugung eines sprunghaften Vskositätsanstiegs eines Gels in einem Temperaturbereich zwischen 18°C und 25°C, wobei der sprunghafte Viskositätsanstieg innerhalb von 2°C erfolgt und wobei außer Poloxamer 407 kein weiteres Poloxamer enthalten ist.

## Claims

1. Antiseptic gel based on poloxamer 407, comprising octenidine and/or polihexanide as active ingredients, **characterized in that** the gel contains between 16% by weight and less than 20% by weight poloxamer 407, but no additional poloxamer from the group consisting of poloxamer 108, poloxamer 182, poloxamer 188, poloxamer 237, poloxamer 331, and poloxamer 338, the gel having an increase in viscosity of at least 20 Pa s in the temperature range of between 18°C and 25°C within a range of 2°C.

2. Antiseptic gel according to any of the preceding claims, **characterized in that** further water and glycerine are contained as components.

3. Antiseptic gel according to any of the preceding claims, **characterized in that** the octenidine and/or polihexanide contents range between 0.01 % by weight and 2% by weight.

4. Antiseptic gel according to any of the preceding claims, **characterized in that** the octenidine and/or polihexanide contents range between 0.05% by weight and less than 0.1% by weight.

5. Antiseptic gel according to any of the preceding claims, **characterized in that** there are contained between 7% by weight and 15% by weight glycerine.

6. Antiseptic gel according to any of the preceding claims, **characterized in that** there are contained at least 58% by weight water.

7. Antiseptic gel according to any of the preceding claims, **characterized in that** it contains no additional poloxamer but poloxamer 407.

8. Antiseptic gel according to any of the preceding claims, **characterized in that** it consists of poloxamer 407, octenidine and/or polihexanide, glycerine and water.

9. A process for preparing an antiseptic gel according to any of the preceding claims, **characterized in that** the preparation is carried out under aseptic conditions.

10. Use of octenidine or polihexanide for the preparation of an antiseptic gel on the basis of poloxamer for the treatment of wounds, wherein the gel contains between 16% by weight and less than 20% by weight poloxamer 407, but no additional poloxamer from the group consisting of poloxamer 108, poloxamer 182, poloxamer 188, poloxamer 237, poloxamer 331, and poloxamer 338 and the gel has an increase in viscosity of at least 20 Pa s in the temperature range of between 18°C and 25°C within a range of 2°C.

11. Use of a mixture of 16% by weight to 20% by weight poloxamer 407, 7 - 15% by weight glycerine and more than 58% by weight water to create a rapid increase in viscosity of a gel in a temperature range of between 18°C and 25°C, wherein the rapid increase in viscosity takes place within 2°C and wherein no additional poloxamer but poloxamer 407 is contained.

## Revendications

1. Gel antiseptique à base de poloxamère 407, comprenant de l'octénidine et/ou de la polyhexanide comme principes actifs, **caractérisé en ce que** le gel contient entre 16 % en poids et moins de 20 % en poids de poloxamère 407, mais ne contient pas d'autre poloxamère provenant du groupe constitué des suivants : poloxamère 108, poloxamère 182, poloxamère 188, poloxamère 237, poloxamère 331 et poloxamère 338, le gel présentant une augmentation de la viscosité d'au moins 20 Pa.s dans la plage de températures comprise entre 18 °C et 25 °C, dans une plage de 2 °C.

2. Gel antiseptique selon la revendication précédente, **caractérisé en ce qu'**il contient de l'eau et du glycérol comme constituants.

3. Gel antiseptique selon l'une quelconque des revendications précédentes, **caractérisé en ce que** la teneur en octénidine et/ou en polyhexanide se situe entre 0,01 % en poids et 2 % en poids.

4. Gel antiseptique selon l'une quelconque des revendications précédentes, **caractérisé en ce que** la teneur en octénidine et/ou en polyhexanide se situe entre 0,05 % en poids et moins de 0,1 % en poids.

5. Gel antiseptique selon l'une quelconque des revendications précédentes, **caractérisé en ce qu'**il contient entre 7 % en poids et 15 % en poids de glycérol.

6. Gel antiseptique selon l'une quelconque des revendications précédentes, **caractérisé en ce qu'**il contient au moins 58 % en poids d'eau.

7. Gel antiseptique selon l'une quelconque des revendications précédentes, **caractérisé en ce qu'**il ne contient aucun autre poloxamère en dehors du poloxamère 407.

8. Gel antiseptique selon l'une quelconque des revendications précédentes, **caractérisé en ce qu'**il est constitué de poloxamère 407, d'octénidine et/ou de polyhexanide, de glycérol et d'eau.

9. Procédé de fabrication d'un gel antiseptique selon l'une quelconque des revendications précédentes, **caractérisé en ce que** l'on réalise la fabrication dans des conditions aseptisées.

10. Utilisation d'octénidine ou de polyhexanide pour fabriquer un gel antiseptique à base de poloxamère pour le traitement de plaies, dans laquelle le gel contient entre 16 % en poids et moins de 20 % en poids de poloxamère 407, mais ne contient pas d'autre poloxamère provenant du groupe constitué des suivants : poloxamère 108, poloxamère 182, poloxamère 188, poloxamère 237, poloxamère 331 et poloxamère 338, et le gel présente une augmentation de la viscosité d'au moins 20 Pa.s dans la plage de températures comprise entre 18 C et 25 °C, dans une plage de 2 °C.

11. Utilisation d'un mélange constitué de 16 % en poids à 20 % en poids de poloxamère 407, de 7 à 15 % en poids de glycérol et de plus de 58 % en poids d'eau pour générer une augmentation brusque de la viscosité d'un gel dans une plage de températures comprise entre 18 °C et 25 °C, l'augmentation brusque de la viscosité se faisant dans une plage de 2 °C et aucun autre poloxamère n'étant contenu en dehors du poloxamère 407.
